# EUROPEAN PATENT APPLICATION

(11) **EP 4 116 434 A1**
(43) Date of publication of application: **11.01.2023**
(21) Application number: 21382615.9
(22) Date of filing: 07.07.2021
(51) Int. Cl.: C12Q 1/6841

(54) **IN VITRO METHOD FOR QUANTIFYING THE CELLULAR CONTENT OF SPECIFIC RNAS**

(71) Applicant: Universidad Autónoma de Madrid, 28049 Madrid (ES)
(72) Inventor: GONZÁLEZ VASCONCELLOS, Iria María, 28049 Madrid (ES); COBOS FERNÁNDEZ, María de los Ángeles, 28049 Madrid (ES); FERNÁNDEZ PIQUERAS, José, 28049 Madrid (ES); SANTOS HERNANDEZ, Javier, 28049 Madrid (ES)
(74) Representative: Hoffmann Eitle

(57) **Abstract**

The present invention refers to an in vitro method for quantifying total cell RNA content, preferably total cell content of long-non-coding RNAs (IncRNA), more preferably total cell content of IncRNA molecules transcribed from telomeric or sub-telomeric sequences or present in telomeric regions, such as TERRA and/or TERC, using a labelled probe and flow cytometry.

## Description

### FIELD OF THE INVENTION

The present invention refers to the biomedical field. Particularly, it refers to an *in vitro* method for quantifying the cellular content of a given RNA in a size independent manner. Specifically, the cellular content of long-non-coding RNA (IncRNAs), preferably total cellular content of IncRNA molecules transcribed from telomeric or sub-telomeric sequences, or present in telomeric regions, such as TERRA and/or TERC, can be quantified according to the present invention.

### PRIOR ART

LncRNAs are defined as non-coding RNAs typically longer than 200 base pairs lacking protein coding ability. They play crucial roles at the epigenetic, transcriptional, post-transcriptional, translational and post-translational levels, including roles as nucleic acid scaffold and chromatin silencers/remodellers. Various IncRNAs serve as biomarkers for the diagnosis and prognosis of various diseases, including cancer.

Specifically, there is a IncRNA transcribed from the sub-telomeric to the telomeric region called Telomere repeat-containing RNA (TERRA), whose levels are altered in cancer cells compared to normal cells and in other diseases associated with telomere shortening such as ICF (immunodeficiency, centromeric region instability, facial anomalies). This IncRNA is characterized by 5'-(UUAGGG)-3' repeats, only transcribed from the C-rich strand and pairing therefore with a C-rich telomeric peptide nucleic acid (PNA) probe. Its quantification remains up to now somehow elusive as the gold standard used for its quantification, the qPCR, can only target those TERRA arising from one chromosomal end at a time. No ubiquitous TERRA quantification has been developed until now able to quantify large numbers of cells, as the RNA FISH can only quantify TERRA foci (either quantifying the number of foci, or the brightness of the PNA signal within the cellular foci and in cell numbers not bigger than 100/200 cells normally and with the restricted resolution given by a microscope). Another IncRNA is the human telomerase RNA component (TERC), a long non-coding RNA that is an essential component of telomerase, which unlike the catalytic protein component (TERT) is ubiquitously expressed in most human terminally differentiated cells and of exceptional importance for malignisation and inflammatory responses. Its telomeric recognition sequence (AAUCCC) will pair with a G-rich telomeric PNA probe.

So, there is an unmet biomedical need of finding a method able to quantify the cellular content of specific RNA molecules in a fast and reliable manner, preferably total cell content of specific IncRNAs, more preferably total cell content of IncRNA molecules transcribed from telomeric or sub-telomeric sequences or present in telomeric regions, such as TERRA and/or TERC. The identification of the cellular content of specific RNA molecules_would be important at clinical level. In fact, the quantification of total cell content of specific IncRNA molecules transcribed from telomeric or sub-telomeric sequences or present in telomeric regions, such as TERRA and/or TERC, could be used for the diagnosis of diseases associated with telomere shortening or dysfunction such as cancer, ICF, or other diseases disclosed in the following references: [Ng, L. J., Cropley, J. E., Pickett, H. A., Reddel, R. R. & Suter, C. M. Telomerase activity is associated with an increase in DNA methylation at the proximal subtelomere and a reduction in telomeric transcription. Nucleic Acids Res. 37, 1152-1159 (2009*)],* [Yehezkel, S., Segev, Y., Viegas-Péquignot, E., Skorecki, K. & Selig, S. Hypomethylation of subtelomeric regions in ICF syndrome is associated with abnormally short telomeres and enhanced transcription from telomeric regions. Hum. Mol. Genet. 17, 2776-2789 (2008*)], [*TERC promotes cellular inflammatory response independent of telomerase. Nucleic Acids Res. 47, 8084-8095 (2019*)]* and *[*Xi, X. et al. RNA Biomarkers: Frontier of Precision Medicine for Cancer. Non-Coding RNA 3, (2017*)].*

The present invention is focused on solving this problem and, consequently, a fast and reliable *in vitro* method for quantifying the cellular content of specific RNA molecules, preferably the cellular content of specific RNA molecules of IncRNA, more preferably the cellular content of specific IncRNA molecules transcribed from telomeric or sub-telomeric sequences or present in telomeric regions, such as TERRA and/or TERC, is herein provided.

### DESCRIPTION OF THE INVENTION

### Brief description of the invention

As explained above, the present invention refers to an *in vitro* method for quantifying the cellular content of a given RNA in a size independent manner, preferably total cell content of specific IncRNAs, more preferably the cellular content of a given IncRNA transcribed from telomeric or sub-telomeric sequences or present in telomeric regions, such as TERRA and/or TERC.

In a specific embodiment, the method of the invention is based on coupling the specificity of a telomeric PNA probe with the cytometer technology to detect and quantify IncRNAs, avoiding RNA degradation and DNA crosstalk in a simple, reliable, specific and fast manner. Thus, the method of the invention is an easy-to-use method, which allows enhanced, specific high-throughput data to achieve conclusive results on IncRNA quantification with broad application in clinical and basic research.

The quantification of IncRNAs, particularly TERRA and/or TERC, has been carried out in the present invention as proof of concept. However, the method herein described could be used for the quantification of total cell content of any RNA molecule, by combining the use of labelled probes in suitable conditions to avoid double stranded DNA denaturation, and possible probe hybridization with the DNA (thus providing suitable conditions for the probe to hybridize only with the RNA and protecting RNA integrity) and flow cytometry.

So, the first embodiment of the present invention refers to an *in vitro* method (hereinafter "method of the invention") for quantifying total cell RNA content in a biological sample which comprises: a) Contacting a labelled probe with the cells to be analysed in a suitable hybridization media; b) Incubating the hybridization mixture at a temperature below 45°C, making sure that the presence of formamide in the hybridization buffer did not contribute to significantly DNA denaturation and therefore telomeric DNA is not unavailable for PNA labelling, thus providing suitable conditions for the probe to hybridize only with the RNA, and protecting RNA integrity; and c) Quantifying total RNA content by flow cytometry.

In a preferred embodiment, the method of the invention is used for analysing, measuring and/or quantifying total IncRNA cellular content.

In a preferred embodiment, the method of the invention is used for analysing, measuring and/or quantifying total cellular IncRNA molecules transcribed from telomeric or sub-telomeric sequences or present in telomeric regions.

In a preferred embodiment, the method of the invention is used for analysing, measuring and/or quantifying total cell content of the IncRNAs TERRA and/or TERC.

In a preferred embodiment, the labelled probe is a fluorescent-labelled probe.

In a preferred embodiment, the fluorescent-labelled probe is a PNA probe.

In a preferred embodiment, the method of the invention is used for analysing, measuring and/or quantifying total cell content of IncRNAs in a biological sample obtained from a subject which comprises: a) Contacting a fluorescent-labelled PNA probe with the biological sample in a suitable hybridization media, b) Incubating the hybridization media at a temperature below 45°C in order to avoid double stranded DNA denaturation and probe hybridization with the DNA, thus providing suitable conditions for the probe to hybridize with the RNA and protecting RNA integrity, and c) Analysing, measuring and/or quantifying total cell IncRNA content by flow cytometry.

In a preferred embodiment, the method of the invention is used for analysing, measuring and/or quantifying total cell content of the IncRNAs TERRA and/or TERC, in a biological sample obtained from a subject, which comprises: a) Contacting a fluorescent-labelled PNA probe with the biological sample in a suitable hybridization media, b) Incubating the hybridization media at a temperature below 45°C in order to avoid double stranded DNA denaturation and probe hybridization with the DNA, thus providing suitable conditions for the probe to hybridize with the RNA and protecting RNA integrity; c) Analysing, measuring and/or quantifying total cell content of the IncRNAs TERRA and/or TERC by flow cytometry.

In a preferred embodiment, the method of the invention comprises a step a) wherein the cell pellet is collected from the biological sample before contacting with the labelled probe.

In a preferred embodiment, the step b) of the method of the invention further comprises the addition of an RNA protecting agent to prevent RNA degradation.

In a preferred embodiment, the biological sample used for implementing the method of the invention is blood.

In a particularly preferred embodiment the method of the invention comprises the following steps: a) Collecting cell pellet, rinse and centrifuge, b) Resuspending cells in one hybridization buffer with the conjugated PNA probe (for improved results an RNA protecting agent is added), c) Incubating all tubes at a low temperature, preferably below 45 °C, making sure DNA denaturation is avoid at all times (the result is improved by with gentle shaking to avoid probe and cellular precipitation at long incubation times), d) After staining, centrifuging samples and washing the pellet by adding a wash buffer, e) Washing/Rinsing by using appropriate buffer with or without detergent, and f) Resuspending in appropriate buffer for flow-cytometry.

The second embodiment of the present invention refers to an *in vitro* method for the diagnosis of diseases associated with telomere shortening or dysfunction (such as cancer or ICF) which comprises analysing, measuring and/or quantifying total cell content of IncRNA molecules transcribed from telomeric or sub-telomeric sequences, or present on the telomeric sequences, such as TERR and/or TERC, by following the method of the invention described above.

For the purpose of the present invention the following terms are defined:
- The term "comprising" means including, but not limited to, whatever follows the word "comprising". Thus, use of the term "comprising" indicates that the listed elements are required or mandatory, but that other elements are optional and may or may not be present.
- The term "consisting of" means including, and limited to, whatever follows the phrase "consisting of'. Thus, the phrase "consisting of" indicates that the listed elements are required or mandatory, and that no other elements may be present.

### Description of the figures

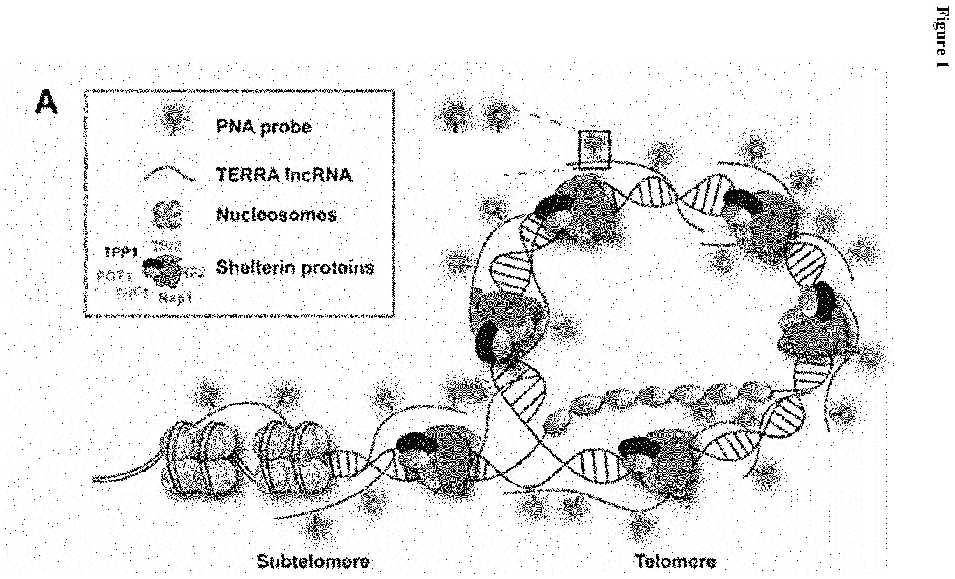

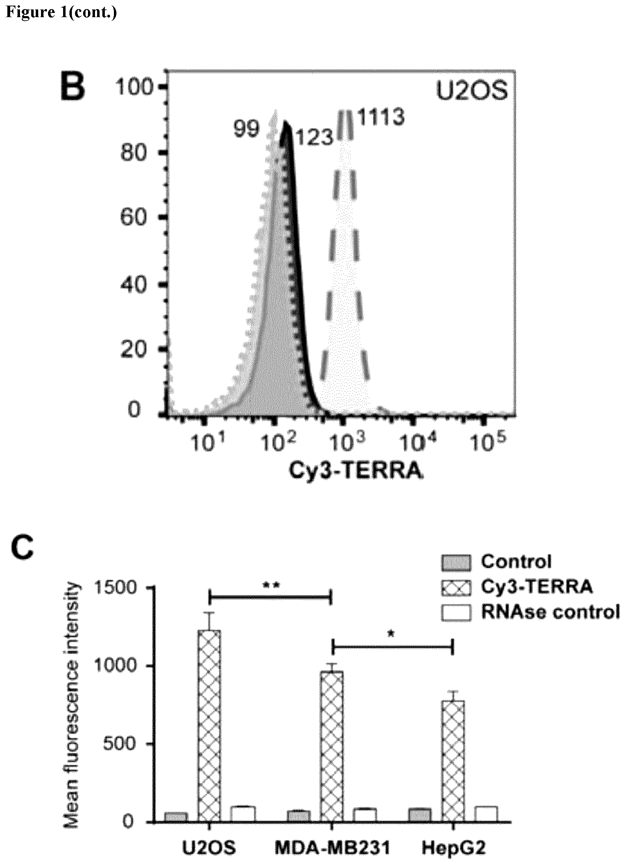

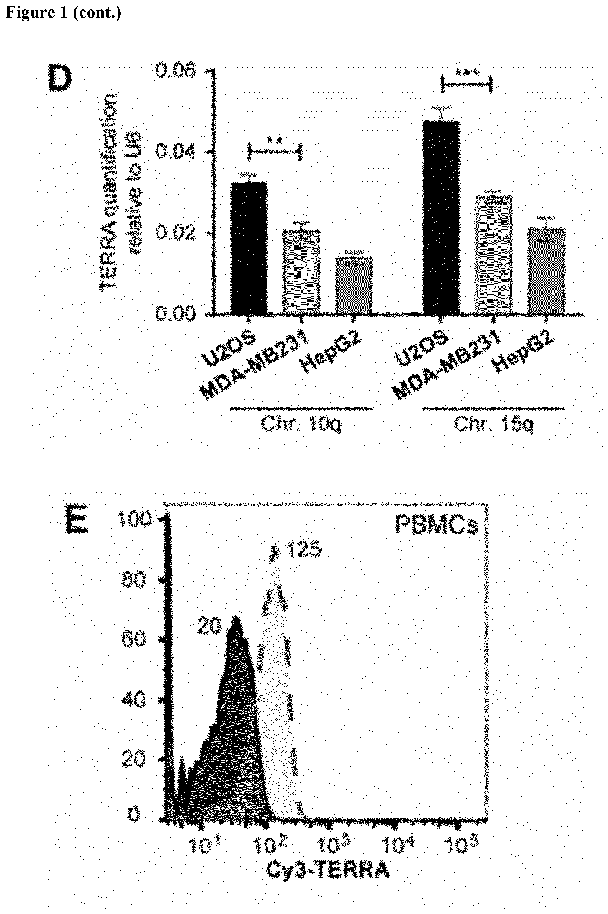

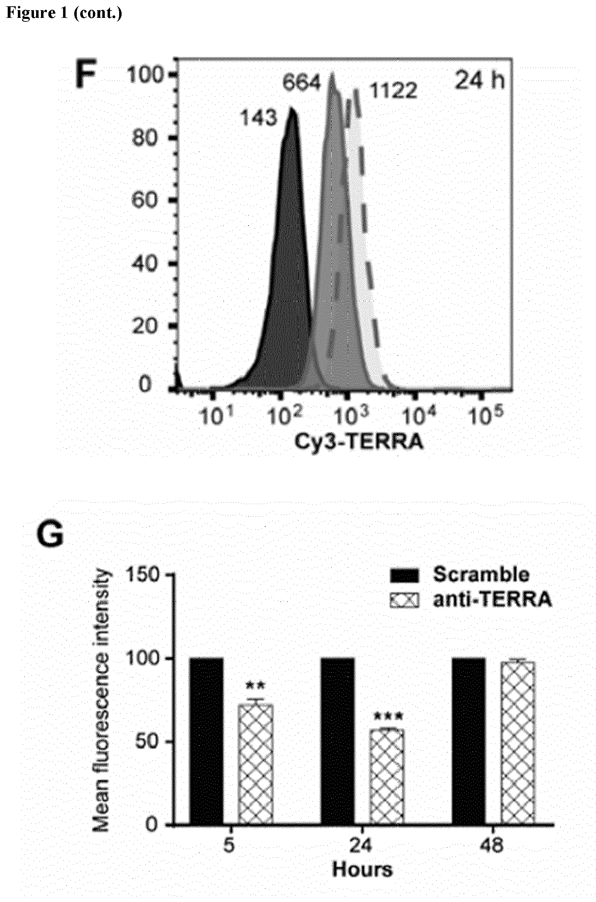

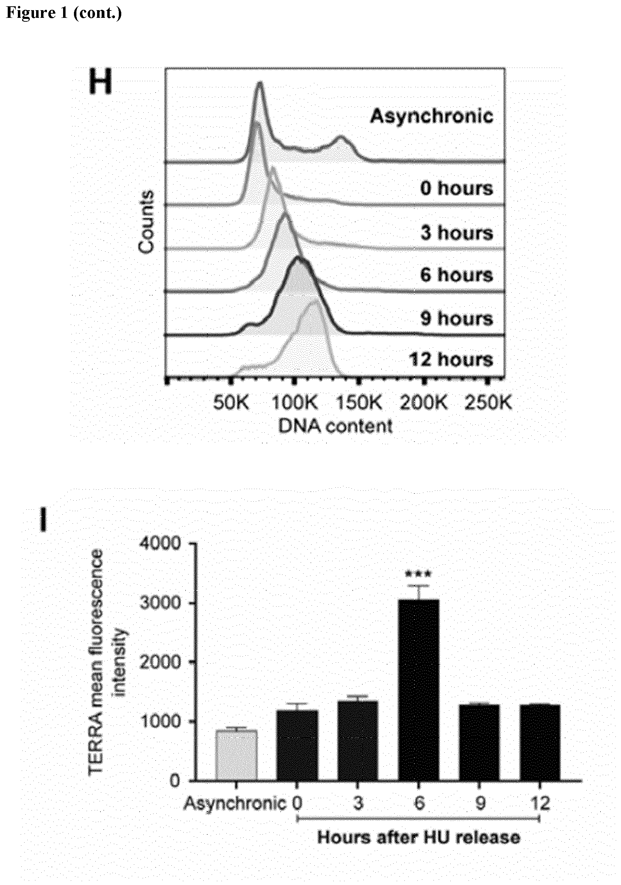

**Figure 1****. PNA-RNA-flow-FISH for TERRA quantification. A)** Scheme of the TelC PNA probe hybridising TERRA IncRNA at telomeric chromatin. **B)** Representative cytometry image of TERRA quantification (long dashed line filled in red peak) using PNA-RNA-flow-FISH versus the unstained control (Black solid line peak) and the stained sample together with an RNase treatment (short dashed line filled with grey peak) in U2OS cells. C) Quantification of 3 independent PNA-RNA-flow-FISH experiments staining TERRA IncRNA on U2OS, MDA-MB231 and HepG2 cell lines. Grey bars show the unstained controls, checked bars show the mean of TERRA quantification and unfilled bars show the RNAse control. Numbers represent the mean fluorescence intensity of 3 independent experiments. ** P ≤ 0.01 and * P ≤ 0.05 using Student's t-test. **D)** Validation of the PNA-RNA-flow-FISH method by quantifying TERRA expression using RT-qPCR on human chromosomes 10q and 15q. Mean of 3 biological replicates ** P ≤ 0.01 and *** P ≤ 0.001 using Student's t-test. **E)** Representative image of PNA-RNA-flow-FISH on PBMCs. Long dashed line filled in red peak represent the mean fluorescence of TERRA versus their unstained control (black solid line peak). Numbers represent the mean fluorescence intensity. **F)** Representative image of the PNA-RNA-flow-FISH on TERRA quantification 24 hours after transient knockdown using GapmeR technology. Black solid line peak represents the unstained control. Long dashed line filled with light red peak represents the scramble control and the solid line filled in dark red represents the cells with TERRA knockdown. Numbers represent the mean fluorescence intensity. **G)** TERRA quantification at different time points after GapmeR transfection in U2OS cells. Bars show the mean of 3 independent experiments. Solid bars are the scramble controls for each time point assayed. Checked bars are the TERRA quantification on knockdown cells. Mean of 3 biological replicates ** P ≤ 0.01 and *** P ≤ 0.001 using Student's t-test. **H)** Representation of the cell cycle distribution over time after HU block and release in U2OS cells. **I)** TERRA quantification on each cell cycle stage after HU block release. TERRA levels increase at a specific point of the S phase (6 hours after release). Results show the mean of 3 independent experiments *** P ≤ 0.001 using Student's t-test.

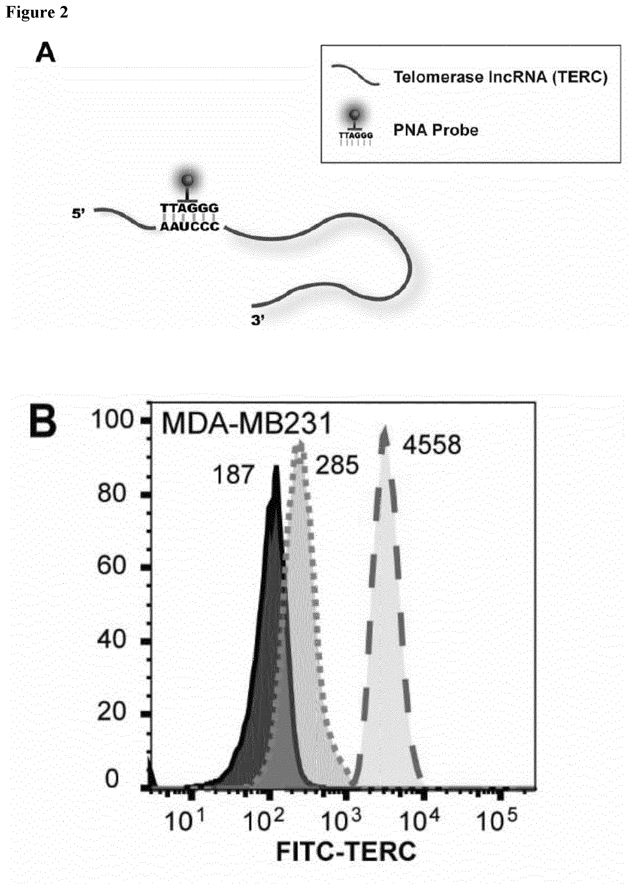

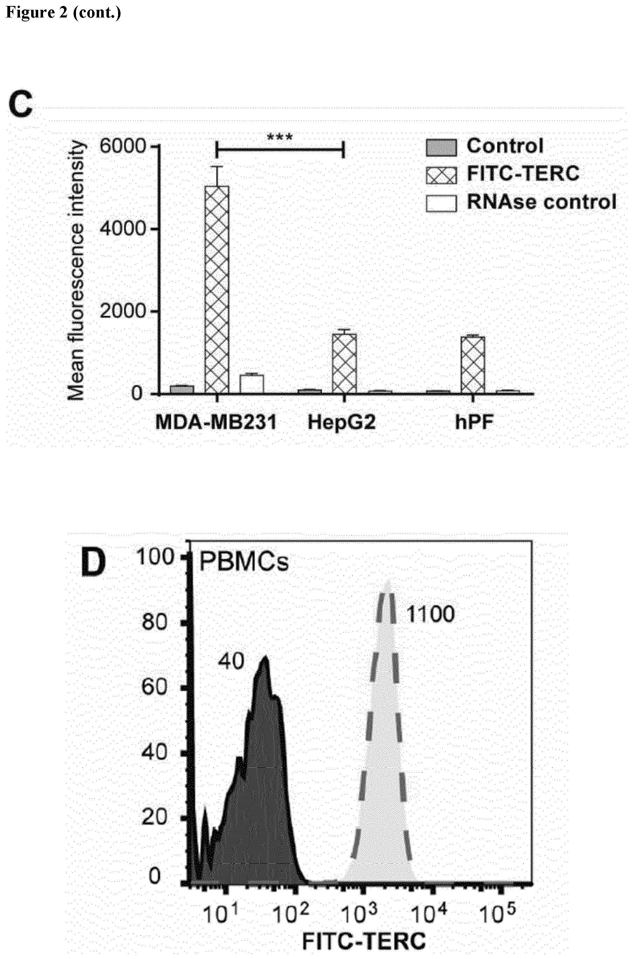

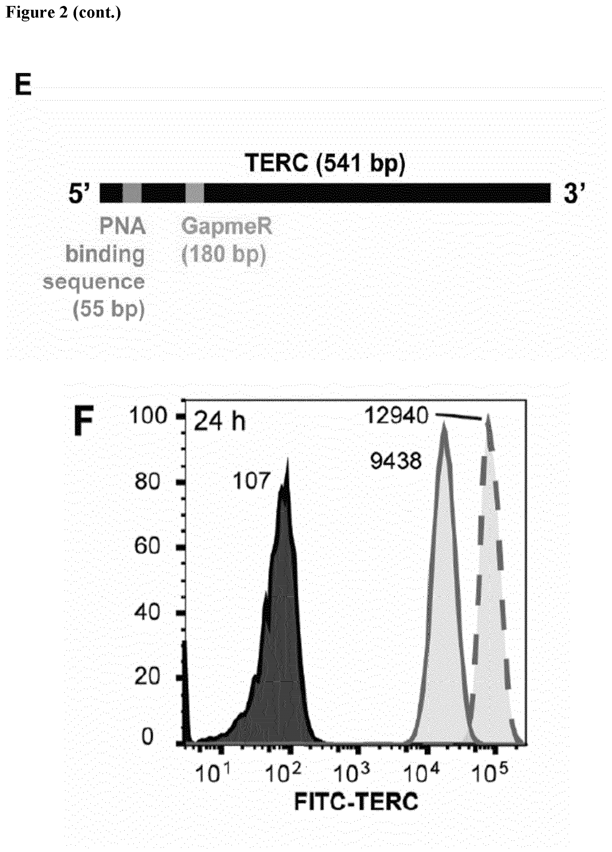

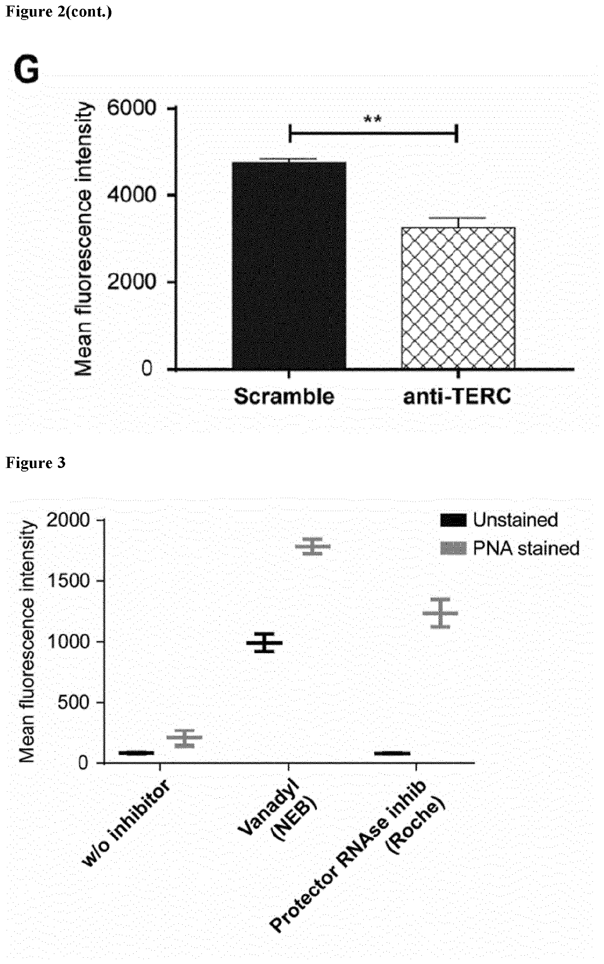

**Figure 2****. PNA-RNA-flow-FISH for TERC quantification. A)** Scheme of the TelG PNA probe hybridising TERC IncRNA at the telomere recognition sequence. **B)** Representative cytometry image of TERC quantification (long dashed line filled in green peak) using PNA-RNA-flow-FISH versus the unstained control (Black solid line peak) and the stained sample together with an RNase treatment (short dashed line filled with grey peak) in MDA-MB231 cells. **C)** Quantification of 3 independent PNA-RNA-flow-FISH experiments staining TERRA IncRNA on MDA-MB231, HepG2 cell lines and human primary fibroblasts (hPF). Grey bars show the unstained controls, checked bars show the mean of TERC quantification and unfilled bars show the RNAse control. Numbers represent the mean fluorescence intensity. *** P ≤ 0.0001 using Student's t-test. **D)** Representative image of PNA-RNA-flow-FISH on PBMCs. Long dashed line filled in green peak represent the mean fluorescence of the TERC versus the unstained control (black solid line peak). Numbers represent the mean fluorescence intensity. **E)** Scheme of the newly developed TERC GapmeR against TERC IncRNA downstream the telomere recognition site. **F)** Representative image of the PNA-RNA-flow-FISH on TERC quantification 24 hours after transient knockdown of the IncRNA using GapmeR technology. Black solid line peak represents the unstained control. Long dashed line filled with light green peak represents the scramble control and the solid line filled with green represents the cells with TERC knockdown. Numbers represent the mean fluorescence intensity. **G)** TERC quantification 24 hours after GapmeR transfection in MDA-MB231 cells. The solid bar is the scramble control. The checked bar is the TERC quantification of the knockdown cells. Mean of 3 biological replicates ** P ≤ 0.01 using Student's t-test.
**Figure 3****. RNAse Inhibitor study.** Two protectors RNAse inhibitors were tested to find a suitable one for the assay. Vanadyl form NEB was discarded due to autofluorescence whilst the RNAse inhibitor from Roche (03335399001) was suitable to protect the RNA and increase the signal without interfering with the methodology used.

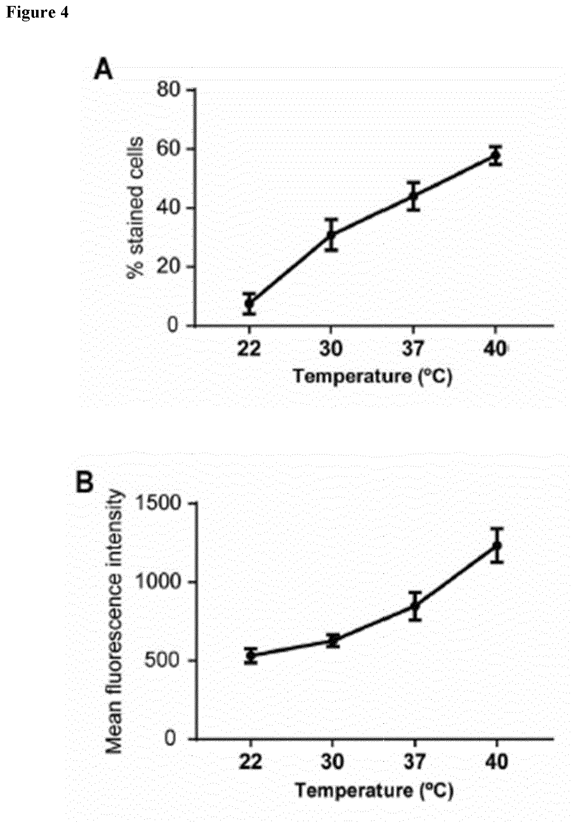

**Figure 4****. Temperature study.** Temperatures from room temperature to 44-45 degrees centigrade were tested in order to establish the best conditions for the assay. **A)** regarding number of cells stained and **B)** mean fluorescence intensity. And making sure the reduction of the melting point due to the formamide in the hybridisation buffer does not infer cross reactions with the DNA.

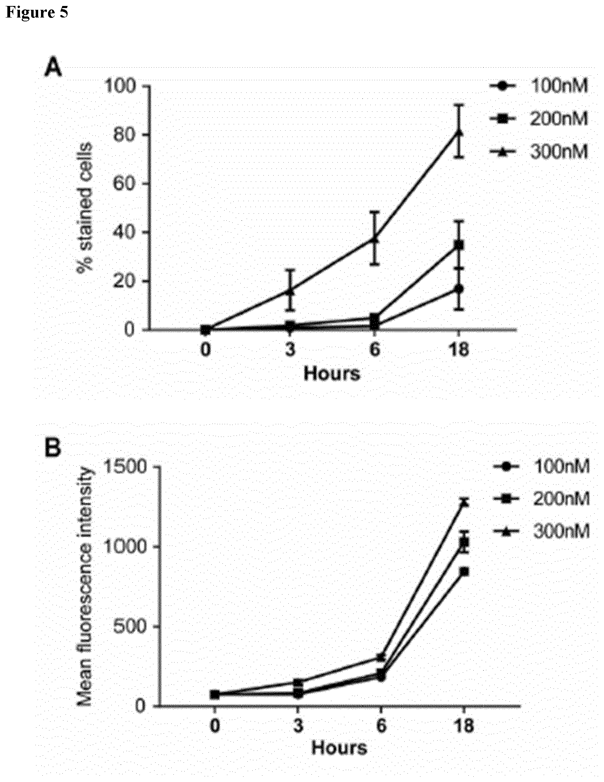

**Figure 5****. PNA hybridization condition study on RNA staining.** Curves depicted with the different conditions assayed, time and concentration wise. **A)** percentage of cells stained at different probe concentrations and at different times after addition of the probe. **B)** Mean fluorescence intensity of cells stained at different probe concentrations and at different times after addition of the probe

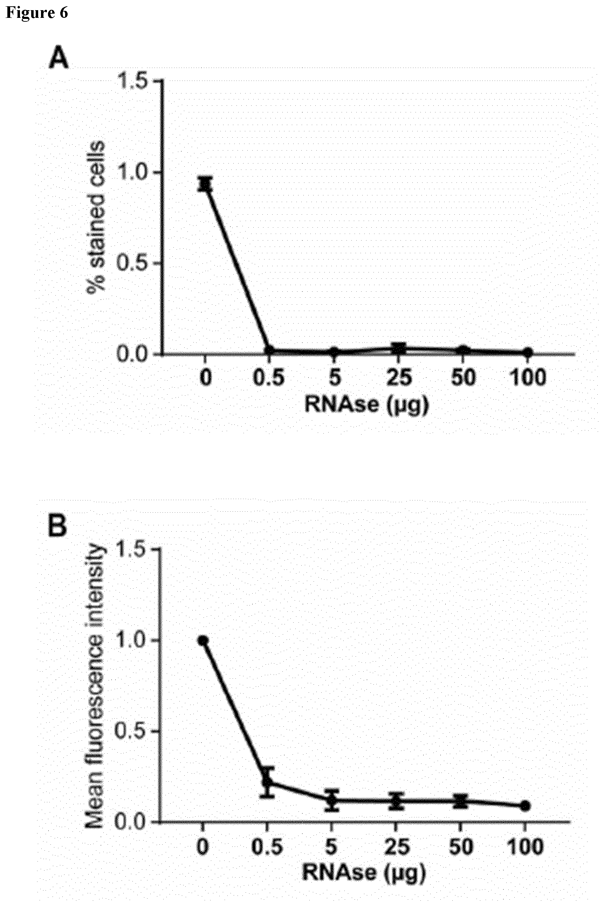

**Figure 6****. RNAse study.** Incubating the cells with the PNA probe and different RNA concentrations allowed us to demonstrate that with 0.5 µg we could almost abolish the PNA signal in terms of **A)** number of cells stained and **B)** mean fluorescence intensity per sample.

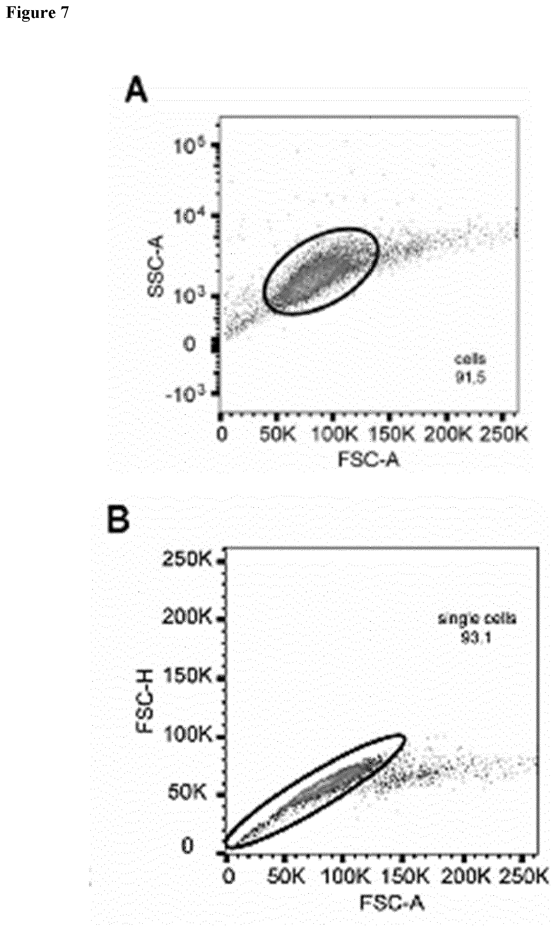

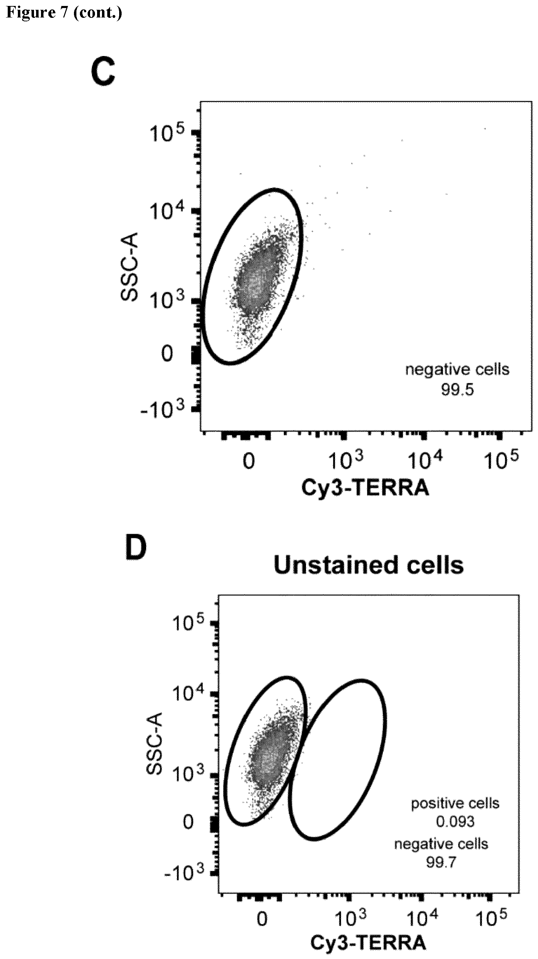

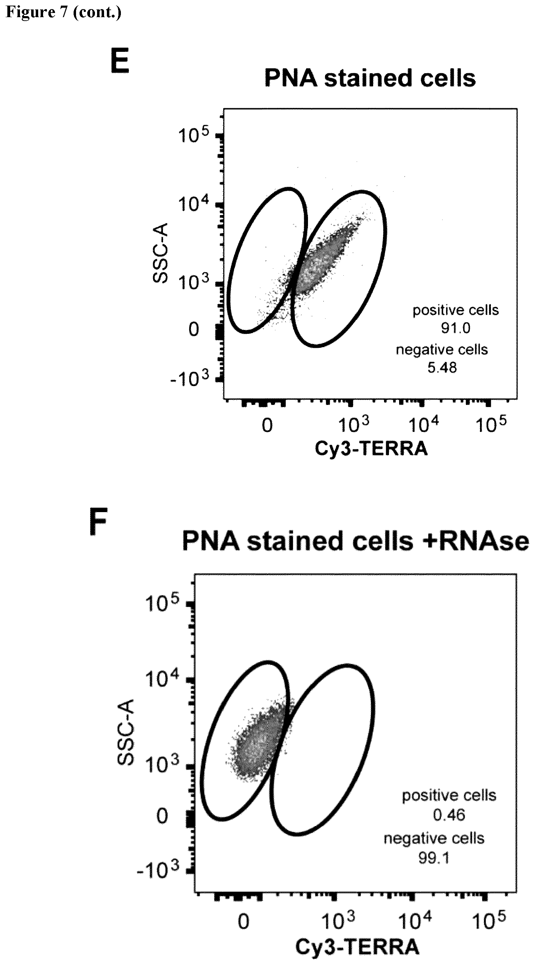

**Figure 7****. Cytometry approach to populations. A)** Alive cells were gated on the SSC-A/FSC-A plot. **B)** single cells were gated in the FSC-H/FSC-A plot. **C)** Negative cells were gated in the SSC-A/FL2 detector. **D)** Gating of negative population in unstained samples. **E)** Gating of the positive stained PNA-TERRA samples. **F)** Gating of the PNA stained co-incubated with RNAse H.

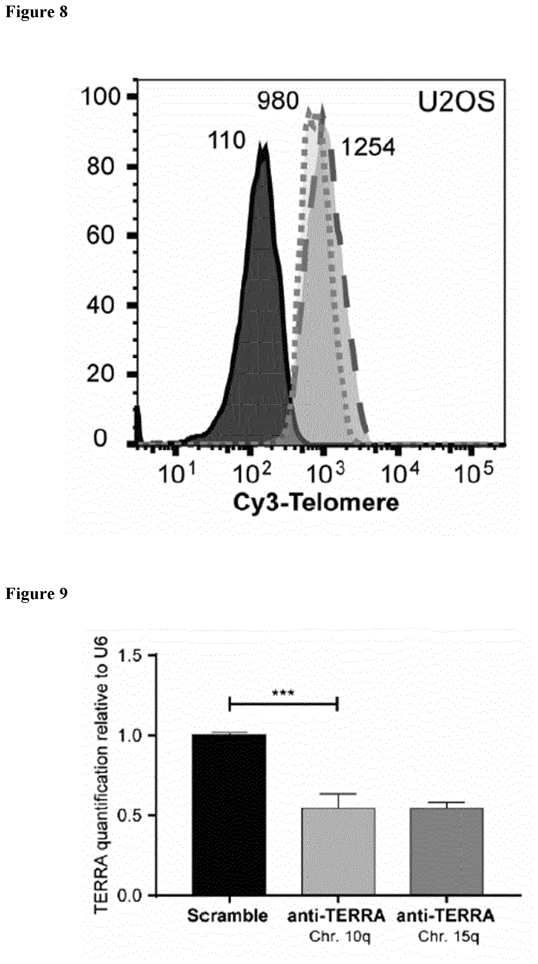

**Figure 8****. Telomere staining in U2OS with the TelC probe.** DNA-Flow FISH. The protocol was initiated with a denaturation for 10 minutes at 85°C. Black peak represents the unstained cells. Red dashed line peak depicts the mean fluorescence of telomeres. Short dashed peak filled in grey is the stained cells with the RNAse treatment. Note that in this case, the RNAse treatment does not affect the staining as DNA is being labelled in this case.
**Figure 9****. qPCR on GapmeR transfected U2OS cells on chromosomes 10q and 15q.** Demonstration of the TERRA reduction after knockdown with the GapmeR technology 24 hours after transfection. *** P ≤ 0.0001 using Student's t-test.

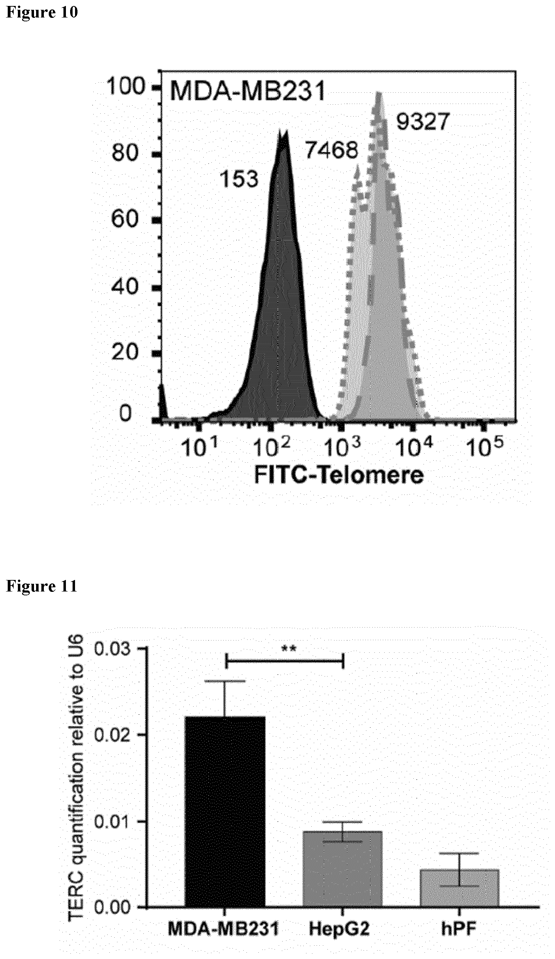

**Figure 10****. Telomere staining in MDA-MB231 with the TelG probe. DNA-Flow FISH.** The protocol was initiated with a denaturation for 10 minutes at 85°C. Black peak represents the unstained cells. Green dashed line peak depicts the mean fluorescence of telomeres. Short dashed peak filled in grey is the stained cells with the RNAse treatment. Note that in this case, the RNAse treatment does not affect the staining as DNA is being labelled in this case.
**Figure 11****. TERC study by qPCR of MDA-MB231, HepG2 and hPF cells.** Results support those obtained using PNA-RNA-flow-FISH. ** P ≤ 0.001 using Student's t-test.

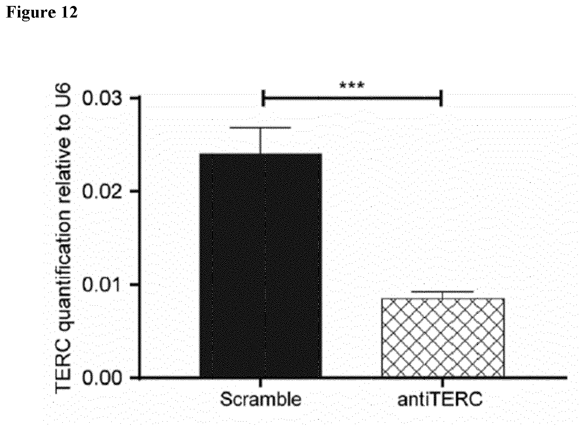

**Figure 12****. TERC study on knockdown MDA-MB231 by qPCR.** Solid bar is the scramble control. Checked bar is the value of the TERC expression 24 hours after TERC GapmeR knockdown. *** P ≤ 0.0001 using Student's t-test.

### Detailed description of the invention

The present invention is illustrated by means of the examples set below, without the intention of limiting its scope of protection

### Example 1. Material and methods

### Example 1.1. Cell culture

All cell lines used in this study (U2OS, HEPG2, MDA-MB231 and hPF) were kept in culture in DMEM (Gibco) with 10% foetal bovine serum and 1% glutamine. Incubated at 37°C in a humidified atmosphere with 5% CO₂. Cells were passaged when reaching confluence by using Trypsin/EDTA. hPF Human primary fibroblasts (hPF) were extracted from a piece of foetal muscle from an autopsy performed in a natural abortion. The participant provided written informed consent in accordance with the Declaration of Helsinki.

### Example 1.2. PNA-RNA-Flow

A step-by-step protocol is herein disclosed. In brief:
PNA labelling: 0,5x10⁶ cells were resuspended in 500 µL hybridisation buffer containing: 70% Formamide (Sigma-Aldrich), 20mmol/L Tris buffer, pH 7, 1µl/ml of the protector RNAse inhibitor (Roche) and 300 mM of PNA probe. The RNAse treatment study was performed by adding into the labelling hybridisation buffer different concentrations of RNAse from bovine pancreas DNAse free (Roche). Incubations were performed overnight at 40 degrees with gentle shaking (300 rpm).
Analysis by flow cytometry: After staining, all cells were washed twice in wash buffer I containing 70% formamide, with 0,1% tween-20 followed by one extra wash in wash buffer II containing PBS with 0,1% tween-20. Cells were resuspended in 500 µL PBS for analysis. Results were obtained in a FACSCanto A Flow Cytometer (Becton Dickinson Biosciences) and analysed with the FlowJo (FlowJo, Ashland, OR) software.

### Example 1.3.DNA-Flow-FISH

PNA labelling: 0,5x10⁶ cells were resuspended in 500 µL hybridisation buffer containing: 70% Formamide (Sigma-Aldrich) and 20mmol/L Tris buffer, pH 7. Denaturation was performed at 80 degrees for 10 minutes followed by the addition of 300mM PNA probe and 50mg of RNAse (Roche) and an overnight incubation at 40 degrees with gentle shaking (300 rpm).

Analysis by flow cytometry: After staining, all cells were washed twice in wash buffer I containing 70% formamide, with 0,1% tween-20 followed by one extra wash in wash buffer II containing PBS with 0,1% tween-20. Cells were resuspended in 500 µL PBS for analysis. Results were obtained in a FACSCanto A Flow Cytometer (Becton Dickinson Biosciences) and analysed with the FlowJo (FlowJo, Ashland, OR) software.

### Example 1.4. Propidium iodide labeling for FACS analysis of the cell cycle

Cells were trypsinised and 1,5 x 10⁶ cells were washed with 1 mL PBS and spun down at 400g for 5 minutes. 1 mL of 70% ethanol (cold) was added drop by drop to the cellular pellet while gently vortexing and left over-night at -20°C. The following day fixed cells were centrifuged at 1500g for 5 minutes. Cellular pellet was washed twice in 1 ml PBS, centrifuged at 400 g for 5 minutes. Clean pellet was then resuspended in 0.5 ml of PI/RNAse staining buffer (BD Pharmingen). Incubate for 30 minutes at room temperature in the dark. Results were obtained in a FACSCanto A Flow Cytometer (Becton Dickinson Biosciences) and analysed with the FlowJo (FlowJo, Ashland, OR) software.

### Example 1.5. TERRA RT-qPCR

Total RNA was extracted with the High Pure RNA Tissue kit (Roche diagnostics). RNA was treated with RNase-Free DNase (Qiagen) for 1 hour, cleaned and concentrated with RNA Clean & Concentrator^{™} (Zymo Research) with an additional in-column DNase treatment made prior to elution. Specific reverse transcription of TERRA and the U6 control RNA were performed using 10 µM of the TERRA specific oligonucleotide (CCCTAA)₅ or 1µM of the U6 oligonucleotide GAACTCGAGTTTGCGTGTCATCCTTGCGC (SEQ ID NO: 1) respectively.

A qPCR was performed with 3 µl of this newly synthesized cDNA using GoTaq qPCR Master Mix (Promega), in a final volume of 20µl. The following primers were used for the amplification of the human subtelomeric region from chromosome 10q (forward: GAATCCTGCGCACCGAGAT) (SEQ ID NO: 2), reverse (CTGCACTTGAACCCTGCAATAC) (SEQ ID NO: 3). For TERC the primers (forward: TCTAACCCTAACTGAGAAGGGCGTAG) (SEQ ID NO: 4), reverse (GTTTGCTCTAGAATGAACGGTGGAAG) (SEQ ID NO: 5) were used and U6 as housekeeping gene.

### Example 1.6. GapmeR transfection for TERRA/TerC knockdown

Transient *in vitro* knockdown of TERRA and TERC in U2OS and MDA-MB-231 respectively, was achieved using 15 nM screening-grade modified antisense oligonucleotide GapmeRs, (Qiagen, formerly Exiqon) 300.000 U2OS (TelC) or MDA-MB231 (TelG) were seeded in 6 well plates in 2 ml of complete medium. The following day, media was replaced by fresh media before transfection. Cells were transfected with lipofectamine 3000 according to manufacturer's protocol. In brief in one tube 7.5 µl of lipofectamine 3000 were diluted in 125 µl of Opti-MEM medium. In another tube 150pmol of GapmeR (either scramble, anti-TERRA or anti-TERC) were diluted in 125 µl of Opti-MEM medium. Both tubes were mixed 1:1 and incubated 15 minutes at room temperature. These 250 µl of DNA-lipid complex are added drop by drop to the cell culture. Cells were incubated at 37°C in a humidified atmosphere with 5% CO₂ and collected at the desired time points.

Scramble LNA GapmeR scramble :5'CACGTCTATACACCAC 3' (SEQ ID NO: 6); LNA GapmeR anti-TERRA: 5'TAACCCTAACCCTAA 3' (SEQ ID NO: 7) and LNA GapmeR anti-TERC: 5'CTCTAGAATGAACGGT 3' (SEQ ID NO: 8) (Quiagen).

### Example 1.7. HU cell cycle block

250.000 cells were seeded in a 6 well plate in 2 ml complete medium. Hydroxyurea stock solution (500mM) was prepared fresh before addition to the culture and filtered sterilised with a 0.2 µm pore size filter. Mix 50 mL of complete medium with 400 µL of filter sterilized HU stock solution for a final HU concentration of 4 mM. Remove medium from all wells except from those needed for defining FACS settings and replace with a freshly prepared 4 mM HU-containing complete medium (2 mL/well). Incubate cells for 24 h in HU-containing medium at 37 °C in a humidified atmosphere with 5% CO2. Remove HU-containing medium from wells and rinse wells twice with pre-warmed 1x PBS (2 mL each time). Add 2 mL of complete medium per well. Collect asynchronous sample and 0 h time point (synchronous). Place remaining wells in the incubator and collect at the desired time points (3, 6, 9 and 12 hours).

### Example 1.8. Step by step PNA-Flow

1) Collect cell pellet. 500.000 cells rinse in PBS and centrifuge 5 minutes at 1500 rpm.
2) Discard supernatant
3) Resuspend cells in 500 µL of hybridization mix containing: 70% formamide (Sigma-Aldrich), 20mmol/L Tris buffer, pH 7 and 40U/ml of the protector RNAse inhibitor (Roche) and 300mM of conjugated PNA probe (Panagene, south Korea). Set up parallel experiments
   - without PNA probe
   - with PNA probe
   - with PNA probe and RNAse
4) Incubate all tubes over night at 40 degrees with gentle shaking (300 rpm)
5) The following morning centrifuge samples at 1500g for 7 minutes
6) Discard supernatant
7) Wash pellet by adding 500 µl of wash buffer I containing: 70% formamide, with 0,1% tween-20
8) Wash for 5 minutes at room temperature. Use shaking, around 700 rpm
9) Centrifuge samples at 1500g for 7 minutes
10) Discard supernatant
11) Repeat steps 7 to 10
12) Wash pellet by adding 500 µl of wash buffer II containing: PBS with 0,1% tween-20
13) Wash for 5 minutes at RT. Use shaking, around 700 rpm
14) Centrifuge samples at 900g for 7 minutes
15) Discard supernatant
16) Resuspend in 500 µl of PBS and transfer into cytometer tubes.

### Example 2. Results

### Example 2.1. lcRNA quantification

To circumvent the limitations of existing methods and quantify specific RNA molecules in a fast, reliable and ubiquitous manner, we developed the PNA-RNA-Flow technique described in the present invention.

To quantify TERRA IncRNA, we used a C-rich PNA telomeric probe (AATCCC)n (Tel C probe, PANAGENE, South Korea) that labels the IncRNA with a fluorochrome, in this study marked with Cy3 **(****Figure 1A****)** and detectable by flow cytometry, in specific hybridisation conditions, so that accurate targeting of the IncRNA is achieved.

Two key points are relevant to achieve a correct IncRNA labelling when conjugating the use of PNA probes and flow-cytometry. On one side, avoiding RNA degradation during the process by using a protector RNAse inhibitor. In this study, two RNA protectors were tested, the Vanadyl Complex (New England Biolabs) and the protector RNAse inhibitor (Roche) and whilst the Vanadyl complex was not useful for this technique due to its own autofluorescence, the protector RNAse inhibitor (Roche) was able to prevent RNA degradation without interfering with the quantification **(****Figure 3****).** On the other side, avoiding DNA denaturation by controlling the temperature during the labelling process not to rise above 40-45 Celsius degrees at any time, making sure that the presence of formamide in the hybridization buffer did not contribute to significantly DNA denaturation, and therefore making sure that telomeric DNA is not unavailable for PNA labelling. We tested different hybridisation temperatures to find out that at 40-45 Celsius degrees, a temperature far from DNA denaturation allowed correct labelling of over 90% of the cells **(****Figure 4A****)** with a high mean fluorescence intensity **(****Figure 4A****).** Over-night labelling with 300mM TelC works best for RNA detection both in regard to number of cells stained **(****Figure 5A****)** as well as the mean fluorescence intensity **(****Figure 5B****).** We also performed an RNAse control to test the effectiveness of the technique by using different RNAse concentrations incubated together with the PNA labelling, and this showed that by adding 0,5 µg of RNAse H (Roche) the PNA signal is almost completely abolished, and higher concentrations of the RNAse completely abolished the PNA signal **(****Figure 6****).**

The method was performed as follows, in brief: We harvested U20S cells to label the TERRA IncRNA by diluting 300mM of the TelC PNA probe (PANAGENE, South Korea) in a hybridisation mix containing formamide. Cells were then analysed in the cytometer, gating single cells and negative from positive populations **(****Figure 7****)** analysed with the blue laser (488nm), with the FL2 detector for Cy3. Unlabelled U2OS cells (autofluorescence levels only) were used as control and showed a fluorescence peak of 123 units (autofluorescence) whilst in Cy3-PNA labelled U2OS the peak was found in the red spectrum at 1113 units of fluorescence intensity. To demonstrate the specificity of the labelling procedure a staining was also performed following a coincubation with PNA probe and RNAse. Results show that the PNA signal was abolished and the fluorescence peak decreased to 99 fluorescence units, close to that of the control unlabelled cells **(****Figure 1B****).** Moreover, the analysis performed in different cell lines (U2OS, MDAMB231 and HepG2) showed different cellular expression of TERRA and inter-cellular differences were observed **(****Figure 1C****)** comparable to that assayed via the gold standard RT-qPCR method on chromosomes 10q and 15q **(****Figure 1D****),** demonstrating the legitimacy of the technique. One of the benefits of this technique when assaying TERRA is that the gold standard for this IncRNA quantification, must be performed in one chromosome at a time, as primers are designed within the chromosome subtelomeric region and therefore in a chromosome specific manner, whilst with PNA-RNA-Flow the ubiquitous TERRA expression levels are assayed independently of which chromosomal end they arose from. In parallel we assayed the telomeres of these cell lines with the same technique and the same TelC probe by opening the DNA strands by an initial DNA denaturation at 85°C for 10 minutes to demonstrate the signal difference between telomeric DNA and TERRA IncRNA. Note that telomeric DNA signal was not sensitive to the RNAse treatment

### (Figure 8).

To assay whether this protocol could be applied to blood samples, and have therefore a broader clinical application, we extracted human peripheral blood mononuclear cells (PBMCs) from healthy donors and apply the PNA-RNA-Flow method to TERRA quantification. The signal was detectable with lower TERRA expression levels than those seen in the cell lines yet still discernible from the unstained control **(Figure IE),** demonstrating the resolution power of the technique.

For further reassurance of the technical capacity at determining different IncRNA concentrations and to further proof its effectiveness, we knocked down TERRA in U2OS cells by transiently transfecting the cells with the specific antisense LNA GapmeR technology (Quiagen) to detect a reduction of the TERRA signal 5 hours after transfection, reaching TERRA's lowest expression levels at 24 hours **(****Figure 1F****).** This effect was practically lost 48 hours after the transient transfection as expected and TERRA levels were then detected back to normal, comparable to those seen in cells transfected only with the LNA GapmeR scramble control **(****Figure 1G****)** and with levels ratified by RT-qPCR on chromosomes 10 and 15, 24 hours after transfection **(****Figure 9****).** In fact, previous studies using these GapmeR technology, have measured the remaining TERRA levels after depletion via Norther blot analysis (with detection limited by the technique) and conventional RNA-FISH and foci counting 10, a technique unable to measure the free TERRA unbound to telomeres within the foci. Now, PNA-RNA-Flow can determine the exact amount of IncRNA present in the cells with higher detection specificity and sensitivity.

Next, and knowing that TERRA levels vary throughout the cell cycle11,12 we performed a cell cycle analysis using hydroxyurea (HU) to block the U2OS cells in late G1/ early S phase to later release the block collecting samples at different time points 0, 6, 9 and 12 hours. Throughout this time cells synchronically advanced through the cell cycle from G1 to S phase **(****Figure 1H****).** TERRA levels were assayed at the different time points to find out that TERRA expression increased when comparing HU blocked cells, 0 hours after the release which were sitting mostly at G1, to the asynchronic control. Three hours after the release cells which were entering an early S phase, harboured the same amount of TERRA but those cells harvested 6 hours after the release showed a significant increase in TERRA expression, to then decrease its expression 9 hours after the block release where cells sat on late S phase **(****Figure 1I****)** and the same was detected 12 hours after the release, demonstrating that the technique discriminates between different cellular TERRA levels.

Further proof of the methodology was achieved by targeting a second IncRNA named TERC, one of the two main subunits of telomerase, highly related to telomeres and TERRA and of great importance in the prognosis of human cancer 13,14. The idea was that detection of TERC could be performed by using the telomeric TelG PNA probe (PANAGENE, South Korea) whose G-rich sequence is complementary to the telomere recognition sequence of TERC **(****Figure 2A****).** The TelG PNA probe, labelling with FICT and cells were analysed with the blue laser (488nm), with the FL1 detector, was able to detect a clear signal that presumably targeted TERC with a mean fluorescence intensity increasing from 187 in unlabelled cells to 4558 in the labelled telomerase positive breast cancer cell line MDA-MB231 **(****Figure 2B****).** The signal was abolished by adding RNase to the labelling protocol, decreasing to 285 mean fluorescence intensity **(****Figure 2B****)** demonstrating the selective labelling of RNA molecules. In fact, telomeric DNA staining with the same probe performed after denaturation gave a higher signal intensity insensitive to RNAse treatment **(****Figure 10****).** The signal intensity of TERC was assayed in three different cell lines, two telomerase positive cell lines (MDA-MB231 and HepG2 with different TERC expression 6 and in human primary fibroblasts (hPF). All the cell lines showed significantly different TERC levels among them **(****Figure 2C****).** The gold standard RT-qPCR ratified those differences found among the cell lines in PNA-RNA-Flow **(****Figure 11****).** The study of this IncRNA on PBMCs showed a robust identification of this RNA in blood samples using the PNA-RNA-Flow method **(****Figure 2D****)** demonstrating its effectiveness and potential in clinical procedures.

To demonstrate with certainty that we are targeting TERC we used a newly developed antisense LNA GapmeR (Quiagen) specifically designed against TERC IncRNA located towards the 5-prime end of TERC (180 bp) downstream the telomere recognition site located at 55bp **(****Figure 2E****).** Transiently transfecting MDA-MB231 cells with this GapmeR showed a clear signal reduction of the TERC IncRNA 24 hours after transfection **(****Figures 2F** **and** **G****)** and such difference was confirmed by qPCR **(****Figure 12****),**

## Claims

1. *In vitro* method for quantifying total cell RNA content in a biological sample which comprises:
a. Contacting a labelled probe with the cells to be analysed in a suitable hybridization media,
b. Incubating the hybridization mixture at a temperature below 45 °C in order to avoid double stranded DNA denaturation and possible probe hybridization with the DNA, thus providing suitable conditions for the probe to hybridize only with the RNA, and protecting RNA integrity;
c. Quantifying total RNA content by flow cytometry.

2. Method, according to claim 1, for analysing, measuring and/or quantifying total long-non-coding RNA cellular content.

3. Method, according to any of the previous claims, for analysing, measuring and/or quantifying total cell content of long non-coding RNA molecules transcribed from telomeric or sub-telomeric sequences or present in telomeric regions.

4. Method, according to any of the previous claims, for analysing, measuring and/or quantifying total cell content of the long non-coding RNAs TERRA and/or TERC.

5. Method, according to any of the previous claims, wherein the labelled probe is a fluorescent-labelled probe.

6. Method, according to any of the previous claims, wherein the fluorescent-labelled probe is a peptide nucleic acid (PNA) probe.

7. Method, according to any of the previous claims, for analysing, measuring and/or quantifying total cell content of long-non-coding RNAs in a biological sample obtained from a subject which comprises:
a. Contacting a fluorescent-labelled peptide nucleic acid (PNA) probe with the biological sample in a suitable hybridization media,
b. Incubating the hybridization media at a temperature below 45°C in order to avoid double stranded DNA denaturation and probe hybridization with the DNA, thus providing suitable conditions for the probe to hybridize with the RNA and protecting RNA integrity;
c. Analysing, measuring and/or quantifying total cell content of long-non-coding RNAs by flow cytometry.

8. Method, according to any of the previous claims, for analysing, measuring and/or quantifying total cell content of the long-non-coding RNAs TERRA and/or TERC, in a biological sample obtained from a subject, which comprises:
a. Contacting a fluorescent-labelled peptide nucleic acid (PNA) probe with the biological sample in a suitable hybridization media,
b. Incubating the hybridization media at a temperature below 45°C in order to avoid double stranded DNA denaturation and probe hybridization with the DNA, thus providing suitable conditions for the probe to hybridize with the RNA and protecting RNA integrity;
c. Analysing, measuring and/or quantifying total cell content of the long-non-coding RNAs TERRA and/or TERC by flow cytometry.

9. Method, according to any of the previous claims, wherein the step a) comprises collecting the cell pellet from the biological sample before contacting with the labelled probe.

10. Method, according to any of the previous claims, wherein the step b) further comprises the addition of an RNA protecting agent to prevent RNA degradation.

11. Method, according to any of the previous claims, wherein the biological sample is blood.

12. *In vitro* method for the diagnosis of diseases associated with telomere shortening or dysfunction which comprises analysing, measuring and/or quantifying total cell content of long-non-coding RNA molecules transcribed from telomeric or sub-telomeric sequences, or present on the telomeric sequences, following the method of any of the claims 1 to 11.

13. Method, according to claim 12, for the diagnosis of a diseases associated with telomere shortening which comprises analysing, measuring and/or quantifying total cell content of the long-non-coding RNA TERR and/or TERC, following the method of any of the claims 1 to 11.
